Europäisches Patentamt

European Patent Office

Office européen des brevets

(19)

(11) EP 0 802 410 B1

(12) **FASCICULE DE BREVET EUROPEEN**

(45) Date de publication et mention
de la délivrance du brevet:
**06.08.2003 Bulletin 2003/32**

(51) Int Cl.7: **G01N 33/18**, G21C 17/022

(21) Numéro de dépôt: **97400785.8**

(22) Date de dépôt: **04.04.1997**

(54) **Procédé de mesure de la concentration en lithium de l'eau de refroidissement d'un réacteur nucléaire**

Verfahren zur Messung des Lithiumgehaltes von Kühlwasser in einem Kernkraftwerk

Method for measuring the lithium content of cooling water in a nuclear power plant

(84) Etats contractants désignés:
**BE CH DE ES GB LI NL SE**

(30) Priorité: **17.04.1996 FR 9604805**

(43) Date de publication de la demande:
**22.10.1997 Bulletin 1997/43**

(73) Titulaire: **Framatome ANP**
**92400 Courbevoie (FR)**

(72) Inventeurs:
 • **Brun, Christian**
  **78500 Sartrouville (FR)**
 • **Long, Antoine**
  **94360 Bry sur Marne (FR)**

(74) Mandataire: **Bouget, Lucien et al**
**Cabinet Lavoix**
**2, Place d'Estienne d'Orves**
**75441 Paris Cédex 09 (FR)**

(56) Documents cités:
**FR-A- 2 616 259      US-A- 4 204 259**

 • **ULTRAPURE WATER, vol. 5, no. 6, 1988, pages 34-38, XP000612991 S. M. PARANJAPE: "PH AND CONDUCTIVITY OF PWR PRIMARY COOLANT BASED ON MEASURED CONCENTRATIONS OF BORON, LITHIUM AND AMMONIA."**

Remarques:
Le dossier contient des informations techniques présentées postérieurement au dépôt de la demande et ne figurant pas dans le présent fascicule.

## Description

L'invention concerne un procédé de mesure de la concentration en lithium de l'eau de refroidissement d'un réacteur nucléaire et en particulier d'un réacteur nucléaire refroidi par de l'eau sous pression.

Les réacteurs nucléaires qui utilisent de l'eau sous pression comme fluide de refroidissement comportent un circuit appelé circuit primaire dans lequel circule l'eau de refroidissement sous pression, de manière à prélever de la chaleur dans le coeur du réacteur nucléaire disposé à l'intérieur de la cuve et à refroidir les assemblages du coeur, l'eau de refroidissement sous pression assurant ensuite l'échauffement et la vaporisation d'eau alimentaire dans les générateurs de vapeur disposés sur le circuit primaire.

L'eau sous pression circulant dans le circuit primaire est utilisée non seulement comme fluide de refroidissement du réacteur nucléaire mais également comme un moyen pour régler le flux de neutrons dans le coeur du réacteur nucléaire. Dans ce but, on introduit dans l'eau sous pression de refroidissement du réacteur nucléaire, du bore sous forme d'acide borique.

Au cours d'un cycle de production du réacteur nucléaire, c'est-à-dire pendant le fonctionnement du réacteur nucléaire entre deux rechargements en combustible, la concentration en bore de l'eau de refroidissement varie d'environ 2000 ppm (parties par million) à une valeur pratiquement nulle. On compense ainsi la diminution de la réactivité du combustible. L'eau de refroidissement du réacteur nucléaire, en particulier du fait qu'elle renferme une certaine proportion d'acide borique, est susceptible de produire une certaine corrosion des surfaces métalliques avec lesquelles elle vient en contact dans le circuit primaire. Les produits de corrosion formés qui sont transportés par l'eau de refroidissement et qui traversent le coeur du réacteur deviennent radio-actifs et sont susceptibles de se déposer dans certaines parties du circuit primaire du réacteur nucléaire. Ces produits de corrosion radio-actifs sont donc en partie responsable de l'irradiation reçue par les personnels d'exploitation et d'entretien de la centrale nucléaire qui sont amenés à intervenir sur le circuit primaire, pendant les périodes d'arrêt du réacteur nucléaire.

Pour diminuer la quantité de produits de corrosion radio-actifs formés dans le circuit primaire, il est connu de maintenir le pH de l'eau de refroidissement à une valeur constante telle que le pouvoir corrosif de l'eau de refroidissement soit maintenu à un niveau faible. On diminue ainsi l'intensité du rayonnement radio-actif provenant du circuit primaire et la dose absorbée par les personnels d'entretien du réacteur nucléaire. Dans ce but, il est connu de neutraliser partiellement l'acide borique en injectant dans le circuit primaire une base telle que la lithine (LiOH). Du fait que la teneur en acide borique de l'eau de refroidissement varie au cours du cycle de production du réacteur nucléaire, il est nécessaire de faire varier la concentration en lithine pour maintenir le pH à une valeur sensiblement constante.

On est ainsi amené à effectuer des ajustements de la teneur en lithine de l'eau de refroidissement pour amener cette teneur à une valeur déterminée en fonction de la concentration en bore dans l'eau de refroidissement.

Les ajustements de la teneur en lithine sont réalisés soit en ajoutant de la lithine à l'eau de refroidissement, soit en déminéralisant l'eau de refroidissement pour diminuer la teneur en lithine.

Ces ajustements sont déterminés en fonction des résultats de la mesure de la teneur en lithine ou encore de la concentration en lithium dans l'eau de refroidissement primaire du réacteur nucléaire. En fait, les ajustements sont effectués à partir de valeurs moyennes de la concentration en lithium obtenues à partir de plusieurs mesures successives, de manière à éviter de déclencher un processus d'ajustement inutile, dans le cas d'une variation instantanée non significative de la teneur en lithium.

Il est connu, par le FR-A-2.616.259, de mesurer en continu la concentration en lithium de l'eau de refroidissement du circuit primaire d'un réacteur nucléaire à eau sous pression, en prélevant un échantillon d'eau de refroidissement dans le circuit primaire, en mesurant la conductivité électrique de cet échantillon puis en déterminant la concentration de lithium par corrélation linéaire entre la conductivité électrique de l'eau de refroidissement et la concentration en lithium.

Ce procédé de mesure selon l'art antérieur permet de déterminer la concentration en lithium avec un précision satisfaisante et de fournir des valeurs de cette concentration qui sont significatives, lorsque le réacteur fonctionne en régime stationnaire. Toutefois, après certains transitoires de fonctionnement du réacteur nucléaire, par exemple après une phase d'augmentation ou de diminution rapide de la puissance du réacteur, il s'avère que le procédé de mesure est inapproprié et fournit des valeurs qui ne correspondent plus à la réalité.

Le but de l'invention est donc de proposer un procédé de mesure de la concentration en lithium de l'eau de refroidissement d'un réacteur nucléaire à eau sous pression, circulant à l'intérieur du circuit primaire du réacteur et contenant de l'acide borique introduit dans l'eau de refroidissement pour le réglage de la réactivité du coeur du réacteur et de l'hydroxyde de lithium introduit pour le réglage du pH, dans lequel on prélève un échantillon d'eau de refroidissement dans le circuit primaire, on mesure la conductivité de l'échantillon, on détermine la valeur de la concentration en bore de l'eau de refroidissement et on calcule la concentration en lithium à partir de la valeur mesurée de la conductivité électrique et de la valeur de la concentration en bore, ce procédé permettant de déterminer de manière précise et fiable, quel que soit le mode de fonctionnement du réacteur nucléaire, la concentration en lithium instantanée du fluide de refroidissement.

Dans ce but, on calcule la concentration en lithium [Li⁺] par la relation donnée dans la partie caractérisante de la revendication 1.

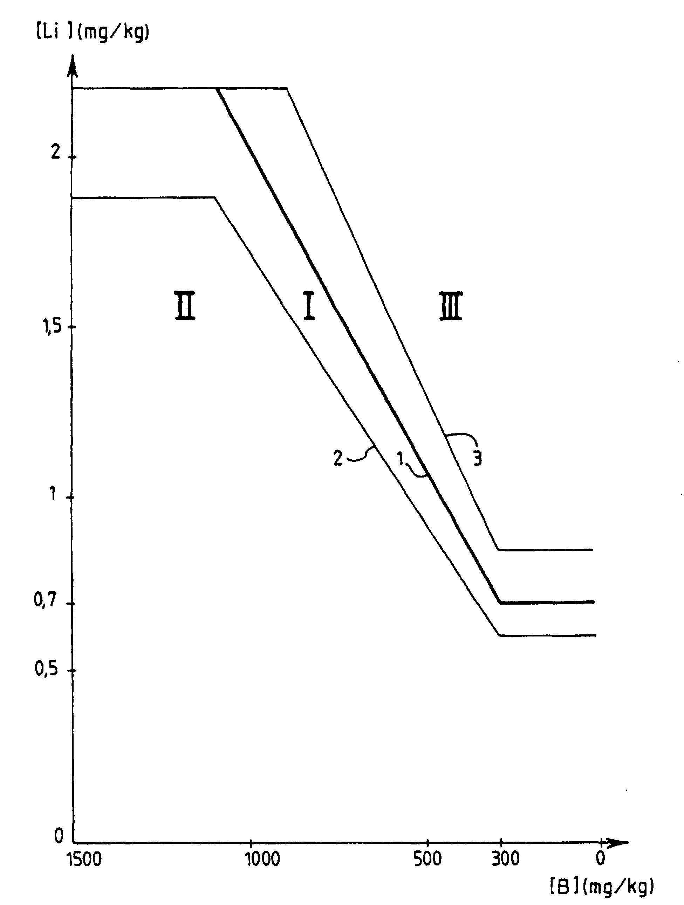

Afin de bien faire comprendre l'invention, on va maintenant décrire, à titre d'exemple non limitatif, en se référant à la figure jointe en annexe, un mode de réalisation du procédé suivant l'invention.

La figure unique est un diagramme donnant la courbe de variation de la teneur en lithium en fonction de la teneur en bore de l'eau de refroidissement d'un réacteur et le principe du réglage de la teneur en lithine de l'eau de refroidissement.

Sur la figure unique, on a représenté une courbe de réglage 1 donnant la valeur de référence de la concentration en lithium de l'eau de refroidissement du réacteur nucléaire en fonction de la concentration en bore de l'eau de refroidissement, pour des valeurs de cette concentration en bore diminuant de 1500 mg/kg à une valeur pratiquement nulle.

Les valeurs de référence de la concentration en lithium données par la courbe 1 et constituées par des portions de droites représentent la concentration qu'il est nécessaire de viser lors des ajustements par addition de lithine dans l'eau de refroidissement ou par déminéralisation de l'eaéu de refroidissement.

On a également tracé, de part et d'autre de la courbe donnant la valeur de référence de la concentration en lithium, deux courbes 2 et 3 constituées également par des portions de droite délimitant un domaine I dans lequel on n'effectue pas d'ajustement de la teneur en lithium, l'écart entre la valeur de la teneur en lithium et la valeur de référence étant considéré comme suffisamment faible. On évite ainsi d'effectuer des ajustements de la teneur en lithine avec une trop grande fréquence pendant le fonctionnement du réacteur nucléaire.

Chacun des points de fonctionnement à l'intérieur de la zone I correspond à des teneurs en acide borique et en lithium de l'eau de refroidissement qui permettent de maintenir le pH de l'eau de refroidissement à une valeur sensiblement constante permettant d'éviter une corrosion importante du circuit primaire.

De part et d'autre de la zone I, les courbes 2 et 3 délimitent une zone inférieure II et une zone supérieure III.

Lorsqu'on observe la présence d'un point de fonctionnement dans la zone II qui constitue une zone interdite, cette présence traduit une concentration en lithium ou en lithine trop faible dans l'eau de refroidissement et il est nécessaire d'ajouter de la lithine LiOH dans l'eau de refroidissement, de manière à ramener le point de fonctionnement dans la zone I.

Lorsqu'on détecte la présence du point de fonctionnement dans la zone III qui est une zone interdite, cette présence traduit une concentration en lithine ou en lithium trop élevée dans l'eau de refroidissement et il est nécessaire de faire passer de l'eau de refroidissement prélevée dans le circuit primaire dans un déminéraliseur à lit cationique permettant de diminuer la concentration en lithium de l'eau de refroidissement. Ce réglage est effectué en mettant en service le déminéraliseur à lit cationique du circuit volumétrique et chimique du réacteur dans lequel circule l'eau de refroidissement prélevée dans le circuit primaire. On peut ainsi faire revenir le point de fonctionnement de la zone III à la zone I.

Les courbes représentées sur la figure correspondent à un fonctionnement normal en puissance du réacteur nucléaire au cours duquel la teneur en bore de l'eau de refroidissement diminue de manière constante.

La valeur de référence de la teneur en lithium donnée par la courbe 1 reste constante et égale à une valeur un peu supérieure à 2 mg/kg tant que la teneur en bore reste supérieure à peu près à 1000 mg/kg. La valeur de référence de la teneur en lithium décroît ensuite de manière linéaire jusqu'à une valeur de l'ordre de 0,7 mg/kg lorsque la teneur en bore passe de 1000 à environ 300 mg/kg. La valeur de référence de la concentration en lithium reste par la suite constante à une valeur de 0,7 mg/kg, lorsque la teneur en bore diminue jusqu'à une valeur pratiquement nulle.

La teneur en bore de l'eau de refroidissement peut être déterminée à chaque instant, à partir des quantités d'acide borique ou d'eau pure ajoutées dans le circuit primaire pendant le fonctionnement du réacteur nucléaire. Cette concentration peut également être mesurée.

Selon la technique connue de l'art antérieur, la concentration en lithium est déterminée à partir d'une mesure de conductivité effectuée sur un échantillon d'eau prélevée dans le circuit primaire du réacteur nucléaire. En réalité, on effectue plusieurs prélèvements et plusieurs mesures de conductivité successives, de manière à en déduire une valeur moyenne de la concentration en lithium, sur une certaine période.

La détermination de la concentration en lithium à partir de mesures de conductivité électrique est suffisamment précise et suffisamment significative et peut être utilisée pour effectuer des ajustements de manière à maintenir le point de fonctionnement dans la zone autorisée, dans le cas où le réacteur nucléaire fonctionne suivant un régime stationnaire. En cas de transitoire, la détermination de la teneur en lithium par des mesures de conductivité électrique n'est plus suffisamment fiable pour permettre un réglage satisfaisant, à une valeur constante, du pH de l'eau de refroidissement.

Selon l'invention, on détermine la concentration en lithium de l'eau de refroidissement à partir d'une mesure de conductivité et de la valeur de la concentration en bore de l'eau de refroidissement. Comme indiqué plus haut, cette valeur de la concentration instantanée en bore de l'eau de refroidissement peut être obtenue à partir des quantités d'acide borique et d'eau ajoutées dans le circuit primaire ou par une mesure.

La concentration en lithium est donnée par la relation (1) indiquée ci-dessous :

$$[Li^+] = \frac{10^3 \times M_{Li}}{\lambda_{Li}} \left\{ \frac{\lambda}{\rho} - \lambda_H [H^+] - \lambda_{OH} \frac{Kw}{[H^+]} - \lambda_B K_1 \frac{[BH]}{[H^+]} - \lambda_{B_2} K_2 \frac{[BH]^2}{[H^+]} - \lambda_{B_3} K_3 \frac{[BH]^3}{[H^+]} \right\} + \beta \quad (1)$$

Dans cette relation :

- [Li$^+$] est la concentration en lithium de l'eau de refroidissement en mg/kg
- $\lambda$ est la conductivité électrique de l'eau de refroidissement en µS/cm. La valeur de ce paramètre est mesurée.
- $M_{Li}$ est la masse atomique du lithium en g
- $\rho$ est la masse volumique de l'eau de refroidissement en kg/dm$^3$
- Les constantes Kw, $K_1$, $K_2$ et $K_3$ sont les constantes d'équilibre des réactions de dissociation de l'eau et de l'acide borique renfermant les ions B(OH)$_4^-$, B$_2$(OH)$_7^-$ et B$_3$(OH)$_{10}^-$, respectivement, ces constantes d'équilibre étant exprimées en Moles/kg
- Les constantes $\lambda_H$, $\lambda_{OH}$, $\lambda_{Li}$, $\lambda_B$, $\lambda_{B2}$, $\lambda_{B3}$, sont les conductivités limites équivalents de chacun des ions indiqués en indice. Ces constantes sont exprimées en µS/cm. Ces constantes sont définies à toute température de manière qu'on peut adapter leur valeur à la température de mesure. Les ions indiqués B, B$_2$ et B$_3$ correspondent aux ions présents dans l'acide borique indiqués ci-dessus.
- $\beta$ est un terme correctif représentatif de la présence éventuelle d'impuretés dans l'eau de refroidissement ; ce terme est exprimé en mg/kg.
- [BH] représente la concentration en acide borique non dissocié qui peut être calculée à partir des teneurs en bore et en lithium par la formule donnée ci-dessous :

$$[BH] \approx 10^{-3} x \frac{[BT]}{M_B} - \frac{[Li]est}{M_{Li}} \tag{2}$$

Dans la formule (2),
[BT] correspond à la teneur en bore de l'eau de refroidissement exprimée en mg/kg.
[Li]$_{est}$ représente la concentration estimée en lithium de l'eau de refroidissement en mg/kg.
$M_B$ et $M_{Li}$ sont les masses atomiques du bore et du lithium exprimées en g.
Dans un premier temps, la valeur de [Li]$_{est}$ est déterminée par la relation :

$$[Li]_{est} = 0,1 \ x \ \lambda \tag{3}$$

- [H$^+$] représente la concentration en ions hydrogène dans l'eau de refroidissement ; cette valeur est calculée à partir de la mesure de conductivité $\lambda$, de la teneur en bore [BT] et d'une première estimation de la teneur en lithium [Li$^+$], par résolution de l'équation du second degré donnée ci-dessous :

$$(\lambda_H - \lambda_{Li})[H^+]^2 - \frac{\lambda}{\rho}[H^+] + (\lambda_{Li} + \lambda_{OH})Kw + (\lambda_{Li} + \lambda_B)K_1[BH] + (\lambda_{Li} + \lambda_{B_2})K_2[BH]^2 + (\lambda_{Li} + \lambda_{B_3})K_3[BH]^3 = 0 \quad (4)$$

de laquelle on déduit la valeur de H :

$$[H^+] = \frac{0,5}{(\lambda_H - \lambda_{Li})}\left[\frac{\lambda}{\rho} - \sqrt{\left(\frac{\lambda}{\rho}\right)^2 - 4 \cdot (\lambda_H - \lambda_{Li})\left\{(\lambda_{Li} + \lambda_{OH})Kw + (\lambda_{Li} + \lambda_B)K_1[BH] + (\lambda_{Li} + \lambda_{B_2})K_2[BH]^2 + (\lambda_{Li} + \lambda_{B_3})K_3[BH]^3\right\}}\right] \quad (5)$$

Pour une température de 25°C qui correspond à la température d'analyse visée de l'échantillon :

$$
\begin{aligned}
- K_w &= 10^{-14} &&(M \times kg^{-1})^2 \\
- K_1 &= 10^{-9,24} &&(M \times kg^{-1}) \\
- K_2 &= 10^{-9,14} &&(M \times kg^{-1})^{-1} \\
- K_3 &= 10^{-7,93} &&(M \times kg^{-1})^{-1} \\
- \lambda'_{B^-} &= 40.10^3 &&\mu S \times cm^{-1} \times eq^{-1} \times l \qquad (6)\\
- \lambda'_{B2} &= 22.10^3 &&\mu S \times cm^{-1} \times eq^{-1} \times l \\
- \lambda'_{B3} &= 16.10^3 &&\mu S \times cm^{-1} \times eq^{-1} \times l \\
- \lambda'_{H^+} &= 350.10^3 &&\mu S \times cm^{-1} \times eq^{-1} \times l \\
- \lambda'_{OH^-} &= 198.10^3 &&\mu S \times cm^{-1} \times eq^{-1} \times l \\
- \lambda'_{Li} &= 35,7.10^3 &&\mu S \times cm^{-1} \times eq^{-1} \times l
\end{aligned}
$$

La concentration en ions H+ de l'eau de refroidissement et donc le pH est donné par l'équation :

$$
[H^+] = \frac{0,5}{311300}\left[\frac{\lambda}{\rho} - \sqrt{\left(\frac{\lambda}{\rho}\right)^2 - 4 \times 311300\left\{236700\, kw + 78700\, K_1[BH] + 60700\, K_2[BH]^2 + 54700\, K_3[BH]^3\right\}}\right] \quad (7)
$$

La teneur en lithium est alors donnée par la relation :

$$
[Li]ppm = \frac{7000}{38700}\left\{\frac{\lambda}{\rho} - 350000 \times [H^+] - \frac{198000 \times 10^{-14}}{[H^+]} - \frac{40000 \times 0^{-9,24}}{[H^+]}[BH] - \frac{22000 \times 10^{-9,14}}{[H^+]}[BH]^2 \right.
$$
$$
\left. - \frac{16000 \times 10^{-7,93}}{[H^+]}[BH]^3\right\} \quad (8)
$$

Pour obtenir une valeur plus précise de la teneur en lithium, il est possible d'effectuer une itération en remplaçant la valeur estimée de la concentration en lithium par la valeur obtenue par le calcul.

Cependant, il s'est avéré que cette itération n'était généralement pas nécessaire pour obtenir une valeur suffisamment précise de la teneur en lithium dans l'eau de refroidissement du réacteur nucléaire.

La température dont il faut tenir compte pour déterminer la valeur des constantes d'équilibre et des conductivités limites est la température de l'échantillon d'eau de refroidissement prélevé sur le circuit primaire par un circuit particulier appelé circuit d'échantillonnage nucléaire. La température de l'échantillon peut être considérée comme sensiblement constante, du fait que les conditions de fonctionnement du circuit d'échantillonnage sont constantes au cours du temps.

Toutefois, il faut tenir compte du fait que la conductivité électrique de l'eau de refroidissement du réacteur dépend de la température.

L'influence de la température sur la conductivité intervient de façon très complexe, du fait de la modification de la densité de l'eau, de l'évolution de la conductivité spécifique de chaque ion présent dans le milieu et de l'évolution des différentes constantes d'équilibre des réactions mises en jeu.

Les lois qui gouvernent l'évolution des constantes d'équilibre et des conductivités équivalentes des ions sont connues. Ces lois ne sont pas des lois de variations linéaires avec la température mais des lois en 1/T qu'il est nécessaire d'utiliser, afin de prendre en compte l'effet de la température.

Une première méthode consiste à réaliser une thermostatisation de la cellule de mesure de conductivité, afin de limiter les corrections de température dans un domaine le plus limité possible. On limite ainsi les erreurs dues aux variations de température. On réalisera une thermostatisation de la cellule de mesure de conductivité, de manière que la température θ soit toujours dans l'intervalle 23°C < θ < 27°C, ce domaine de température de faible amplitude encadrant la valeur de 25°C qui est la température utilisée pour réaliser les mesures.

Une seconde méthode pour tenir compte du paramètre température consiste à mesurer de manière très précise la température de l'eau de refroidissement et à déterminer les différentes constantes à la température de mesure selon des relations du type donné ci-dessous :

$$K_i = A_i/T + B_i$$

$$\overset{.}{\lambda_i} = C_i/T + D_i \tag{9}$$

On intègre les valeurs obtenues dans le calcul du pH et de la teneur en lithium à partir des formules données ci-dessus.

Le procédé de mesure de la concentration en lithium selon l'invention qui a été décrit ci-dessus permet d'obtenir, à partir de la conductivité électrique de l'eau de refroidissement du réacteur nucléaire et de la teneur en bore, une valeur précise de la teneur en lithium de l'eau de refroidissement, pour des concentrations en bore allant de 5 à 2500 mg/kg et des concentrations de lithium de 0,2 à 3,5 mg/kg dans l'eau de refroidissement, dans la mesure où la concentration molaire en bore est supérieure à celle du lithium. Ces intervalles de la teneur en bore et de la teneur en lithium pour lesquels la mesure reste précise et fiable recouvrent très largement les intervalles rencontrés habituellement pour le réglage de pH de l'eau de refroidissement d'un réacteur nucléaire, comme il apparaît en particulier sur la figure.

Le procédé suivant l'invention peut être mis en oeuvre en utilisant des moyens connus de l'homme de métier pour mesurer la conductivité électrique et pour déterminer la teneur en bore.

Pour la mesure de la conductivité électrique, on utilisera de préférence une cellule de mesure thermostatée dont la température peut être maintenue à une valeur aussi proche que possible de 25°C.

La teneur en bore est déterminée en pratique par mesure.

L'invention s'applique à la mesure de la concentration en lithium et au réglage du pH de l'eau de refroidissement d'un réacteur nucléaire de type quelconque.

## Revendications

1.    Procédé de mesure de la concentration en lithium de l'eau de refroidissement d'un réacteur nucléaire, circulant à l'intérieur du circuit primaire du réacteur et contenant de l'acide borique introduite dans l'eau de refroidissement pour le réglage de la réactivité du coeur du réacteur et de l'hydroxyde de lithium introduit pour le réglage du pH, procédé dans lequel on prélève un échantillon d'eau de refroidissement dans le circuit primaire, on mesure la conductivité électrique de l'échantillon, on détermine la valeur de la concentration en bore de l'eau de refroidissement et on calcule la concentration en lithium à partir de la valeur mesurée de la conductivité, électrique et de la valeur de la concentration en bore [BT], **caractérisé par le fait qu'**on calcule la concentration en lithium [Li$^+$] d'après la relation

$$[Li^+] = \frac{10^3 x M_{Li}}{\lambda'_{Li}} \left\{ \frac{\lambda}{p} - \lambda'_H[H^+] - \lambda'_{OH}\frac{kw}{[H^+]} - \lambda'_B K_1 \frac{[BH]}{[H^+]} - \lambda'_{B2} K_2 \frac{[BH]^2}{[H^+]} - \lambda'_{B3} K_3 \frac{[BH]^3}{[H^+]} \right\} + \beta$$

dans laquelle :

-    [Li$^+$] est la concentration en lithium de l'eau de refroidissement en mg/kg ;
-    $\lambda$ est la conductivité électrique de l'eau de refroidissement en uS/cm ;
-    $M_{Li}$ est la masse atomique du lithium en g ;
-    p est la masse volumique de l'eau de refroidissement en kg/dm$^3$ ;
-    Les constantes Kw, $K_1$, $K_2$ et $K_3$ sont les constantes d'équilibre des réactions de dissociation de l'eau et de l'acide borique renfermant les ions B(OH)$_4$, B$_2$(OH)$_7$ et B$_3$(OH)$_{10}$, respectivement ;

Les constantes $\lambda'_H$, $\lambda'_{OH}$, $\lambda'_{Li}$, $\lambda'_B$, $\lambda'_{B2}$, $\lambda'_{B3}$ sont les conductivités limites équivalents de chacun des ions indiqués en indice exprimées en uS/cm ;

$\beta$ est un terme correctif exprimé en mg/kg.

[BH] représente la concentration en acide borique non dissocié qui peut être calculée par la formule donnée ci-dessous :

$$[BH] \approx 10^{-3} \times \frac{[BT]\,[Li]_{est}}{M_B\,M_{Li}}$$

[BT] correspond à la teneur en bore de l'eau de refroidissement exprimée en mg/kg ;

$[Li]_{est} = 0,1 \times \lambda$ (concentration estimée en lithium en mg/kg) ;

$M_B$ et $M_{Li}$ sont les masses atomiques du bore et du lithium exprimées en g , et

$[H^+]$ représente la concentration en ions hydrogène donnée par la formule :

$$[H^+] = \frac{0,5}{(\lambda_H - \lambda_{Li})}\left[\frac{\lambda}{\rho} - \sqrt{\left(\frac{\lambda}{\rho}\right)^2 - 4 \times (\lambda_H - \lambda_{Li})\left((\lambda_H + \lambda_{OH})kw + (\lambda_H + \lambda_B)K_1[BH] + (\lambda_H + \lambda_{B_2})K_2[BH]^2 + (\lambda_H + \lambda_{B_3})K_3[BH]^3\right)}\right]$$

**2.** Procédé suivant la revendication 1, **caractérisé par le fait qu'**on détermine le pH de la solution par la formule :

$$[H^+] = \frac{0,5}{(\lambda_H - \lambda_{Li})}\left[\frac{\lambda}{\rho} - \sqrt{\left(\frac{\lambda}{\rho}\right)^2 - 4 \times (\lambda_H - \lambda_{Li})\left((\lambda_H + \lambda_{OH})kw + (\lambda_H + \lambda_B)K_1[BH] + (\lambda_H + \lambda_{B_2})K_2[BH]^2 + (\lambda_H + \lambda_{B_3})K_3[BH]^3\right)}\right]$$

**3.** Procédé suivant la revendication 1, **caractérisé par le fait qu'**on mesure la conductivité de l'échantillon à une température de 25°C et qu'on utilise pour le calcul de la concentration en lithium la forme simplifiée suivante.

$$[Li]ppm = \frac{7000}{38700}\left\{\frac{\lambda}{\rho} - 350000 \times [H^+] - \frac{198000 \times 10^{-14}}{[H^+]} - \frac{40000 \times 10^{-9,24}}{[H^+]}[BH] - \frac{22000 \times 10^{-9,14}}{[H^+]}[BH]^2 \right.$$
$$\left. - \frac{16000 \times 10^{-7,93}}{[H^+]}[BH]^3\right\}$$

**4.** Procédé suivant la revendication 2, **caractérisé par le fait qu'**on mesure la conductivité électrique de l'échantillon à une température de 25°C et qu'on utilise, pour calculer le pH de l'eau de refroidissement, la formule simplifiée suivante :

$$[H^+] = \frac{0,5}{311300}\left[\frac{\lambda}{\rho} - \sqrt{\left(\frac{\lambda}{\rho}\right)^2 - 4 \times 311300\left(236700\,kw + 78700\,K_1[BH] + 60700\,K_2[BH]^2 + 54700\,K_3[BH]^3\right)}\right]$$

**5.** Procédé suivant l'une quelconque des revendications 1 à 4, **caractérisé par le fait qu'**on utilise une cellule de mesure thermostatée pour déterminer la conductivité électrique de l'échantillon, la température $\theta$ de l'échantillon étant comprise entre 23 et 27°C.

## Patentansprüche

**1.** Verfahren zur Messung des Lithiumgehaltes von Kühlwasser in einem Kernreaktor, bei dem das Kühlwasser innerhalb des Primärkreislaufes zirkuliert, und zur Steuerung der Reaktivität des Reaktorkerns Borsäure sowie Lithiumhydroxid zur pH-Steuerung enthält, wobei dem Kühlwasser innerhalb des Primärkreislaufes eine Probe entnommen wird, die elektrische Leitfähigkeit der Probe gemessen wird, der Borgehalt des Kühlwassers ermittelt wird, und die Lithiumkonzentration, ausgehend von der gemessenen elektrischen Leitfähigkeit und an Hand des Borgehaltes [BT] berechnet wird, wobei die Lithiumkonzentration [Li$^+$] nach folgendem Verhältnis berechnet wird:

$$[Li^+] = \frac{10^3 \times M_{Li}}{\lambda_{Li}'}\left\{\frac{\lambda}{p} - \lambda_H'[H^+] - \lambda_{OH}'\frac{kw}{[H^+]} - \lambda_B'K_1\frac{[BH]}{[H^+]} - \lambda_{B2}'K_2\frac{[BH]^2}{[H^+]} - \lambda_{B3}'K_3\frac{[BH]^3}{[H^+]}\right\} + \beta$$

wobei:

-  $[Li^+]$ die Lithium-Konzentration des Kühlwassers in mg/kg ist;
-  $\lambda$ die elektrische Leitfähigkeit des Kühlwassers in uS/cm ist;
-  $M_{Li}$ die Atommasse des Lithiums in g ist;
-  p die Volumenmasse des Kühlwassers in kg/dm$^3$ ist;
-  die Konstanten Kw, $K_1$, $K_2$ und $K_3$ ausgleichende Konstanten der Dissoziationsreaktionen des Wassers und der Borsäure sind, die folgende Ionen aufweisen: $B(OH)_4$, $B_2(OH)_7$ und $B_3(OH)_{10}$;

die Konstanten $\lambda_H'$, $\lambda_{OH}'$, $\lambda_{Li}'$, $\lambda_B'$, $\lambda_{B2}'$, $\lambda_{B3}'$ die Grenzleitfähigkeiten sind, äquivalent mit jedem der als Richtwert angegeben Ionen, ausgedrückt in uS/cm;
$\beta$ ein Korrektivglied, ausgedrückt in mg/kg, ist;
[BH] den nicht dissoziierten Borsäuregehalt darstellt, der nach folgender Formel berechnet werden kann:

$$[BH] \approx 10^{-3} \times \frac{[BT]\,[Li]est}{M_B\,M_{Li}}$$

[BT] dem Borgehalt des Kühlwassers, ausgedrückt in mg/kg, entspricht;
$[Li]_{est} = 0,1 \times \lambda$ (geschätzter Lithiumgehalt in mg/kg);
$M_B$ und $M_{Li}$ die Bor- und Lithium-Atommassen, ausgedrückt in g, sind, und
$[H^+]$ die Konzentration in Wasserstoffionen, vorgegeben durch folgende Formel, darstellt:

$$[H^+] = \frac{0.5}{(\lambda_H' - \lambda_{Li}')}\left[\frac{\lambda}{p} - \sqrt{\left(\frac{\lambda}{p}\right)^2 - 4 \times (\lambda_H' - \lambda_{Li}')\left\{(\lambda_{Li}' + \lambda_{OH}')kw + (\lambda_{Li}' + \lambda_B')K_1[BH] + (\lambda_{Li}' + \lambda_{B2}')K_2[BH]^2 + (\lambda_{Li}' + \lambda_{B3}')K_3[BH]^3\right\}}\right]$$

2.  Verfahren gemäß Anspruch 1, **dadurch gekennzeichnet, dass** der pH-Wert der Lösung mit folgender Formel festgestellt wird:

$$[H^+] = \frac{0.5}{(\lambda_H' - \lambda_{Li}')}\left[\frac{\lambda}{p} - \sqrt{\left(\frac{\lambda}{p}\right)^2 - 4 \times (\lambda_H' - \lambda_{Li}')\left\{(\lambda_{Li}' + \lambda_{OH}')kw + (\lambda_{Li}' + \lambda_B')K_1[BH] + (\lambda_{Li}' + \lambda_{B2}')K_2[BH]^2 + (\lambda_{Li}' + \lambda_{B3}')K_3[BH]^3\right\}}\right]$$

3.  Verfahren gemäß Anspruch 1, **dadurch gekennzeichnet, dass** die Leitfähigkeit der Probe bei einer Temperatur von 25 °C gemessen wird, und dass die Berechnung der Lithiumkonzentration nach folgender vereinfachter Formel erfolgt:

$$[Li]ppm = \frac{7000}{38700}\cdot\left\{\frac{\lambda}{p} - 350000 \times [H^+] - \frac{198000 \times 10^{-14}}{[H^+]} - \frac{40000 \times 10^{-9.24}}{[H^+]}[BH] - \frac{22000 \times 10^{-5.14}}{[H^+]}[BH]^2\right.$$
$$\left. - \frac{16000 \times 10^{-7.93}}{[H^+]}[BH]^3\right\}$$

4.  Verfahren gemäß Anspruch 2, **dadurch gekennzeichnet, dass** die elektrische Leitfähigkeit der Probe bei einer Temperatur von 25 °C gemessen wird, und dass zur Berechnung des pH-Werts des Kühlwassers folgende vereinfachte Formel genutzt wird:

$$\left[H^+\right] = \frac{0.5}{311300}\left[\frac{\lambda}{\rho} - \sqrt{\left(\frac{\lambda}{\rho}\right)^2 - 4 \cdot 311300\left(236700\ kw + 78700\ K_1[BH] + 60700\ K_2[BH]^2 + 54700\ K_3[BH]^3\right)}\right]$$

**5.** Verfahren gemäß einem der Ansprüche 1 bis 4, **dadurch gekennzeichnet, dass** zur Feststellung der elektrischen Leitfähigkeit der Probe eine Thermostat-Messzelle eingesetzt wird, wobei die Temperatur θ der Probe zwischen 23 und 27 °C beträgt.

**Claims**

**1.** Process for measuring the concentration of lithium in the cooling water of a nuclear reactor, circulating inside the primary circuit of the reactor and containing boric acid added to the cooling water in order to regulate the reactivity of the reactor core and lithium hydroxide added in order to adjust the pH, wherein a sample of cooling water is taken from the primary circuit, the electrical conductivity of the sample is measured, the value of the concentration of boron in the cooling water is determined and the concentration of lithium is calculated from the electrical conductivity measured and the concentration of boron [BT] measured, **characterised in that** the concentration of lithium [Li$^+$] is calculated according to the equation:

$$[Li^+] = \frac{10^3 \times M_{Li}}{\lambda_{Li}}\left\{\frac{\lambda}{\rho} - \lambda'_H[H^+] - \lambda'_{OH}\frac{kw}{[H^+]} - \lambda'_B K_1\frac{[BH]}{[H^+]} - \lambda'_{B2}K_2\frac{[BH]^2}{[H^+]} - \lambda'_{B3}K_3\frac{[BH]^3}{[H^+]}\right\} + \beta$$

wherein

- [Li$^+$] is the concentration of lithium in the cooling water in mg/kg;
- $\lambda$ is the electrical conductivity of the cooling water in uS/cm;
- $M_{Li}$ is the atomic mass of the lithium in g;
- p is the mass by volume of the cooling water in kg/dm$^3$;
- the constants Kw, $K_1$, $K_2$ and $K_3$ are the equilibrium constants of the dissociation reactions of the water and boric acid containing the B(OH)$_4$, B$_2$(OH)$_7$ and B$_3$(OH)$_{10}$ ions, respectively;
- the constants $\lambda'_H$, $\lambda'_{OH}$, $\lambda'_{Li}$, $\lambda'_B$, $\lambda'_{B2}$, $\lambda'_{B3}$ are the extreme equivalent conductivities of each of the ions indicated by the subscripts expressed in uS/cm;
- $\beta$ is a corrective term expressed in mg/kg.

[BH] denotes the concentration of non-dissociated boric acid which can be calculated by the formula given below:

$$[BH] \approx 10^{-3} \times \frac{[BT]\,[Li]_{est}}{M_B\,M_{Li}}$$

[BT] corresponds to the boron content of the cooling water expressed in mg/kg;
[Li]$_{est}$ = 0.1 x $\lambda$ (estimated lithium concentration in mg/kg);
$M_B$ and $M_{Li}$ are the atomic masses of boron and lithium expressed in g, and
[H$^+$] denotes the concentration of hydrogen ions given by the formula:

$$\left[H^+\right] = \frac{0.5}{(\lambda_H - \lambda_{Li})}\left[\frac{\lambda}{\rho} - \sqrt{\left(\frac{\lambda}{\rho}\right)^2 - 4\cdot(\lambda_H - \lambda_{Li})\left((\lambda_H + \lambda_{OH})kw + (\lambda_H + \lambda_B)K_1[BH] + (\lambda_H + \lambda_{B2})K_2[BH]^2 + (\lambda_H + \lambda_{B3})K_3[BH]^3\right)}\right]$$

**2.** Process according to claim 1, **characterised in that** the pH of the solution is determined by the formula:

$$[H^+] = \frac{0.5}{(\lambda_{Li}^0 - \lambda_H^0)}\left[\frac{\lambda}{\rho} - \sqrt{\left(\frac{\lambda}{\rho}\right)^2 - 4 \times (\lambda_{Li}^0 - \lambda_H^0)\left((\lambda_H^0 + \lambda_{OH}^0)kw + (\lambda_{Li}^0 + \lambda_1^0)K_1[BH] + (\lambda_{Li}^0 + \lambda_2^0)K_2[BH]^2 + (\lambda_{Li}^0 + \lambda_3^0)K_3[BH]^3\right)}\right]$$

**3.** Process according to claim 1, **characterised in that** the conductivity of the sample is measured at a temperature of 25°C and the following simplified formula

$$[Li]ppm = \frac{7000}{38700}\cdot\left\{\frac{\lambda}{\rho} - 350000 \times [H^+] - \frac{188000 \times 10^{-14}}{[H^+]} - \frac{40000 \times 10^{-4.24}}{[H^+]}[BH] - \frac{22000 \times 10^{-5.14}}{[H^+]}[BH]^2\right.$$
$$\left. - \frac{16000 \times 10^{-7.93}}{[H^+]}[BH]^3\right\}$$

is used to calculate the concentration of lithium.

**4.** Process according to claim 2, **characterised in that** the electrical conductivity of the sample is measured at a temperature of 25°C and the following simplified formula

$$[H^+] = \frac{0.5}{311300}\left[\frac{\lambda}{\rho} - \sqrt{\left(\frac{\lambda}{\rho}\right)^2 - 4 \times 311300\left\{236700\,kw + 78700\,K_1[BH] + 60700\,K_2[BH]^2 + 54700\,K_3[BH]^3\right\}}\right]$$

is used to calculate the pH of the cooling water.

**5.** Process according to any one of claims 1 to 4, **characterised in that** a thermostatically controlled measuring cell is used to determine the electrical conductivity of the sample, the temperature θ of the sample being between 23 and 27°C.